(19) Europäisches Patentamt
European Patent Office
Office européen des brevets

(11) Veröffentlichungsnummer: **0 292 954 B1**

(12) # EUROPÄISCHE PATENTSCHRIFT

(45) Veröffentlichungstag der Patentschrift: **05.05.93**

(51) Int. Cl.[5]: **C09K 19/58**, C07D 303/48, C07D 405/12, C07D 407/12, C07D 409/12

(21) Anmeldenummer: **88108381.0**

(22) Anmeldetag: **26.05.88**

Die Akte enthält technische Angaben, die nach dem Eingang der Anmeldung eingereicht wurden und die nicht in dieser Patentschrift enthalten sind.

(54) **Verwendung von optisch aktiven Oxiran−2−carbonsäureestern als Dotierstoffe in Flüssigkristallmischungen, diese enthaltende Flüssigkristallmischungen und neue optisch aktive Oxiran−2−carbonsäureester.**

(30) Priorität: **29.05.87 DE 3718174**

(43) Veröffentlichungstag der Anmeldung:
**30.11.88 Patentblatt 88/48**

(45) Bekanntmachung des Hinweises auf die Patenterteilung:
**05.05.93 Patentblatt 93/18**

(84) Benannte Vertragsstaaten:
**AT BE CH DE ES FR GB IT LI NL SE**

(56) Entgegenhaltungen:
**DE−A− 3 731 619**
**US−A− 4 638 073**

(73) Patentinhaber: **HOECHST AKTIENGESELL-SCHAFT**
**Postfach 80 03 20**
**W−6230 Frankfurt am Main 80(DE)**

(72) Erfinder: **Scherowsky, Günter, Prof. Dr.**
**Winklerstrasse 18B**
**W−1000 Berlin 33(DE)**
Erfinder: **Gay, Jürgen**
**Bismarckstrasse 51A**
**W−1000 Berlin 33(DE)**
Erfinder: **Dübal, Hans−Rolf, Dr.**
**Heuhohlweg 6**
**W−6240 Königstein/Taunus(DE)**
Erfinder: **Escher, Claus, Dr.**
**Amselweg 3**
**W−6109 Mühltal(DE)**
Erfinder: **Hemmerling, Wolfgang, Dr.**
**Billtalstrasse 32**
**W−6231 Sulzbach (Taunus)(DE)**
Erfinder: **Müller, Ingrid, Dr.**
**Am Pfingstbrunnen 1**
**W−6238 Hofheim am Taunus(DE)**
Erfinder: **Ohlendorf, Dieter, Dr.**
**Am Kühlen Grund 4**
**W−6237 Liederbach(DE)**
Erfinder: **Wingen, Rainer, Dr.**
**Rotkäppchenweg 10**
**W−6234 Hattersheim am Main(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Flüssigkristalle haben insbesondere im letzten Jahrzehnt Eingang in verschiedene technische Gebiete gefunden, in denen elektro-optische und Anzeigevorrichtungs-Eigenschaften gefragt sind (z.B. in Uhren-, Taschenrechner- und Schreibmaschinenanzeigen). Diese Anzeigevorrichtungen beruhen auf den dielektrischen Ausrichtungseffekten in den nematischen, cholesterischen und/oder smektischen Phasen der flüssigkristallinen Verbindungen, wobei - verursacht durch die dielektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen, die an sich für die Technik wegen ihrer einzigartigen Eigenschaften sehr vielversprechende chemische Verbindungen sind, eher zu lang. Dieser Nachteil macht sich insbesondere dann bemerkbar, wenn eine große Anzahl von Bildpunkten angesteuert werden muß, wodurch die Herstellungskosten von Geräten, die größere Flächen enthalten, z.B. Videogeräte, Oszillographen oder Fernseh-, Radar-, EDV- oder Schreibautomaten-Bildschirme, zu hoch werden.

Neben den nematischen und cholesterischen Flüssigkristallen haben seit einigen wenigen Jahren in zunehmendem Maß auch optisch aktive smektische Flüssigkristall-Phasen an Bedeutung gewonnen.

Clark und Lagerwall konnten zeigen, daß die Verwendung ferroelektrischer Flüssigkristallsysteme in sehr dünnen Zellen zu elektro-optischen Schalt- oder Anzeigeelementen führt, die im Vergleich mit den herkömmlichen TN ("twisted nematic")-Zellen um bis zu einem Faktor 1000 schnellere Schaltzeiten haben (vgl. z.B. Lagerwall et al. "Ferroelectric Liquid Crystals for Displays" SID Symposium, October Meeting 1985, San Diego, Ca., USA). Aufgrund dieser und anderer günstiger Eigenschaften, z.B. der bistabilen Schaltmöglichkeit und des nahezu blickwinkelunabhängigen Kontrasts, sind FLC's grundsätzlich für die obengenannten Anwendungsgebiete, z.B. über eine Matrixansteuerung, gut geeignet.

Einen anderen elektro-optischen Effekt, der als elektrokliner Effekt bezeichnet wird, zeigen orthogonale chirale smektische Phasen, z.B. $S_A^*$, $S_B^*$, $S_E^*$. Dieser Effekt [S. Garoff und R.B. Meyer, Phys. Rev. Lett. 38, 848 (1977)] besteht in einer feldinduzierten Neigung der Moleküle, deren Neigungswinkel $\theta$ sich proportional zum angelegten Feld ändert. Die Moleküle der orthogonalen Phasen können daher der Feldänderung kontinuierlich folgen, und sie können insbesondere einem Wechselfeld bis zu einer Grenzfrequenz $f_G$ folgen, während ferroelektrische Systeme jeweils beim Erreichen einer bestimmten Feldstärke ihren Neigungswinkel sprunghaft ändern und beibehalten, bis ein entsprechendes gegensinniges Feld angelegt wird (bistabiles Schalten).

Beide Effekte, der ferroelektrische, wie der elektrokline, lassen sich je nach ihren speziellen Eigenschaften zum Bau von elektro-optischen Schalt- und Anzeigeelementen ausnutzen. Man benötigt dazu entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische bzw. elektrokline smektische Phasen induzieren. Die gewünschte Phase soll dabei über einen möglichst großen Temperaturbereich stabil sein.

Zur Erzielung eines guten Kontrastverhältnisses in elektro-optischen Bauelementen ist eine einheitliche planare Orientierung der Flüssigkristalle nötig. Eine gute Orientierung in der $S_A^*$- und $S_C^*$-Phase läßt sich erreichen, wenn die Phasenfolge der Flüssigkristallmischung mit abnehmender Temperatur lautet:

$$\text{Isotrop} \rightarrow N^* \rightarrow S_A^* \rightarrow S_C^* .$$

Voraussetzung ist, daß der pitch (Ganghöhe der Helix) in der N*-Phase sehr groß (größer 10 $\mu$m) oder noch besser völlig kompensiert ist (T. Matsumoto et al., S. 468-470, Proc. of the 6th Int. Display Research Conf., Japan Display, Sept. 30 - Okt. 2, 1986, Tokio, Japan; M. Murakami et al., ibid. S.344 - S.347). Dies erreicht man, indem man zu der chiralen Flüssigkristallmischung, die in der N*-Phase z.B. eine linksdrehende Helix aufweist, einen weiteren optisch aktiven Dotierstoff, der eine rechtsdrehende Helix induziert, in solchen Mengen hinzugibt, daß die Helix gerade kompensiert wird.

In der US-Patentschrift 4,638,073 sind 2,3 Epoxyalkyloxiranether beschrieben, die Phenylbenzoat- oder Biphenyleinheiten enthalten. Die spontane Polarisation dieser Verbindungen ist jedoch nicht hoch genug, um zur Herstellung von FLC-Mischungen mit sehr kurzen Schaltzeiten zu genügen.

Es wurde nun gefunden, daß optisch aktive Oxiran-2-carbonsäureester als Dotierstoffe in geneigt-smektischen Flüssigkristallphasen schon bei geringen Zumischmengen zu kurzen Schaltzeiten und in orthogonal smektischen Flüssigkristallphasen zu hohen elektrischen Koeffizienten führen. Besonders überraschend ist dabei, daß die Ganghöhe (pitch) der durch die Dotierung induzierten Helix in der N*-Phase so klein ist, daß bereits kleinste Zumengen zu einer verdrillten Phase mit gegenläufigem Drehsinn deren

Verdrillung kompensieren können.

Gegenstand der Erfindung ist daher die Verwendung von optisch aktiven Oxiran-2-carbonsäureestern als Dotierstoffe in Flüssigkristallsystemen. Gegenstand der Erfindung sind weiterhin Flüssigkristallsystem, die optisch aktive Oxiran-2-carbonsäureester enthalten sowie neue optisch aktive Oxiran-2-carbonsäureester. Die gemäß der Erfindung einzusetzenden Oxiran-2-carbonsäureester entsprechen der allgemeinen Formel (I)

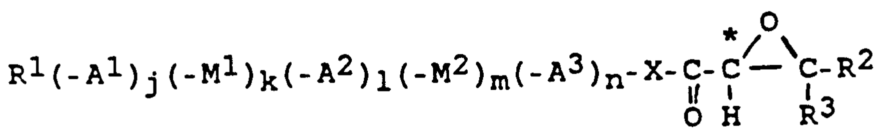 (I)

in der die Symbole und Indices folgende Bedeutung haben:

$R^1$

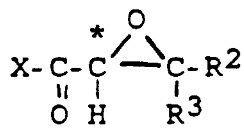

oder ein geradkettiger oder verzweigter Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, eine oder mehrere nicht benachbarte $-CH_2-$ Gruppen durch $-O-$, $-S-$, $-CO-$, $-O-CO-$ und/oder $-CO-O-$ ersetzt sein können, und wobei ein oder mehrere H durch F, Cl oder Br ersetzt sein können.

$R^2$ H oder Alkyl mit $C_1-C_{16}$, oder Cyclohexyl oder Bicyclohexyl, die auch jeweils in Position 4 bzw. 4' durch eine Alkylkette mit 1 bis 16 C-Atomen substituiert sein können,

$R^3$ H

j und l null, 1 oder 2

k und m null oder 1

n null, 1 oder 2

mit folgender Maßgabe: wenn j und/oder 1 = null sind, ist k = null; wenn n = null ist, ist m = null; die Summe j + l + n ist minimal 1 und maximal 3,

$-A^1$, $-A^2$

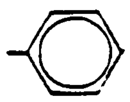, 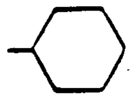, 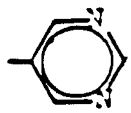, 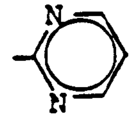,

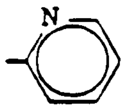, 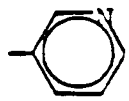, 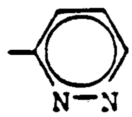, 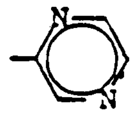,

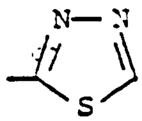, 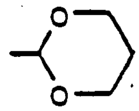, 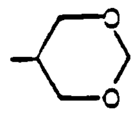, 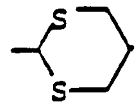,

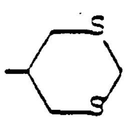, 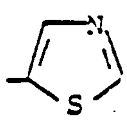, 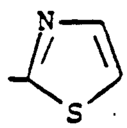, 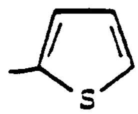,

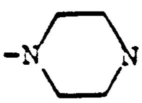,

$-A^3$

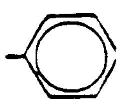, 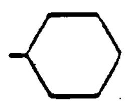, 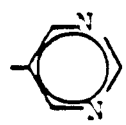, 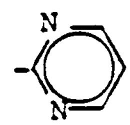,

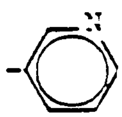, 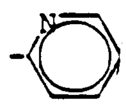, 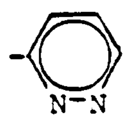

| | |
|---|---|
| $-M^1$, $-M^2$ | $-CO-O$, $-O-CO$, $-CH_2O$, $-OCH_2$ |
| X | O. |

In einer bevorzugten Ausführungsform haben die Symbole in der allgemeinen Formel (I) die folgende Bedeutung:

| | |
|---|---|
| $R^1$ | ein geradkettiger oder verzweigter Alkyl− oder Alkenylrest mit 4 bis 14 C−Atomen, der ein asymmetrisches C−Atom enthalten kann, oder wobei eine $-CH_2-$Gruppe durch $-O-$, $-CO-$ oder $-CO-O-$ ersetzt sein kann, oder wobei ein oder mehrere H durch F ersetzt sein können |
| $R^3$ | H |
| j und l | null oder 1 |
| k, m, n | null oder 1. |

In einer weiteren bevorzugten Ausführungsform werden Oxiran−2−carbonsäureester der allgemeinen Formel (IV) eingesetzt

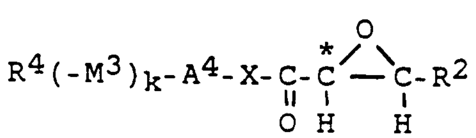 (IV)

worin bedeuten:

$R^4$ einen geradkettigen oder verzweigten Alkyl – oder Alkenylrest mit 6 bis 12 C – Atomen, der ein asymmetrisches C – Atom enthalten kann;

$-M^3$ – O, – S, – O – CO oder – CO

$-A^4$

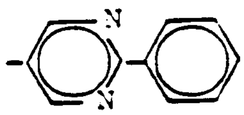,
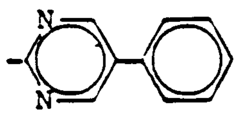,
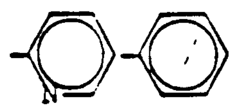,

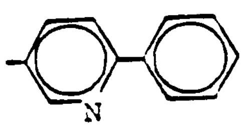,
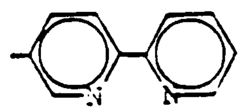,
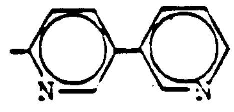,

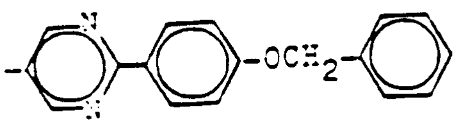,
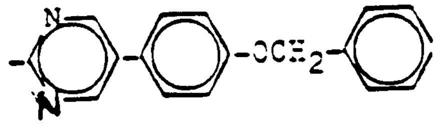,

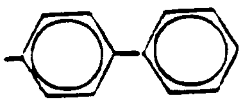,
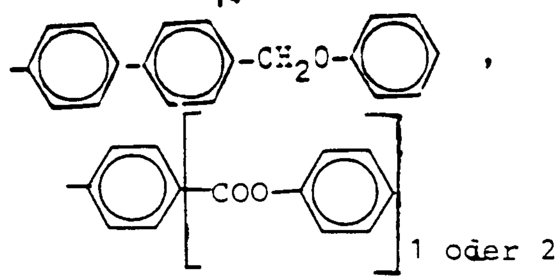

In einer besonders bevorzugten Ausführungsform ist in der allgemeinen Formel (IV) der Rest $R^2$ ungleich H, so daß auch das zweite C – Atom im Oxiranring chiral ist. Zu den neuen Verbindungen der allgemeinen Formel (I), insbesondere (IV) gehören bevorzugt die in den Beispielen namentlich genannten Verbindungen.

Zur Herstellung der Verbindungen der allgemeinen Formel (I) werden mesogene Phenole der allge – meinen Formel (II)

$$R^1(-A^1)_j(-M^1)_k(-A^2)_l(-M^2)_m(-A^3)_n-X-H \qquad (II)$$

mit Derivaten der Oxiran – 2 – carbonsäure der Formel (III)

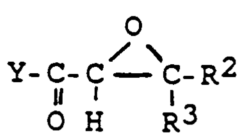

(III)

umgesetzt, wobei Y eine OH – Gruppe oder Halogen bedeutet. Für Y = OH erfolgt die Veresterung mit (II) in Gegenwart von Brönstedt – oder Lewis – Säuren, ggf. in Gegenwart wasserbindender Mittel, oder mit Kondensationsreagentien wie N,N' – Carbonyldiimidazol, Dicyclohexylcarbodiimid oder Azodicarbonsäureester/Triphenylphosphin. Für Y = Halogen erfolgt die Umsetzung mit (II) in Gegenwart von Säurefängern, insbesondere Pyridin oder Triethylamin. Schließlich können auch die Alkali – oder Erdalkalisalze von (II) mit den Säurehalogeniden [(III) mit Y = Halogen] zu (I) umgesetzt werden. Das Reaktionsprodukt kann durch an sich bekannte Maßnahmen, z.B. Umkristallisieren oder chromatographische Trennverfahren, gereinigt werden.

Phenolkörper der Formel (II) sind bekannt. Ebenso sind die Methoden zur Herstellung der Derivate der Oxiran – 2 – carbonsäure (III) bekannt, z.B. Sharpless et al., J. Org. Chem. 46, 3936 (1981); Djerassi et al., J. Am. Chem. Soc. 105, 2408 (1983).

Die Flüssigkristallmischungen gemäß der Erfindung bilden Flüssigkristall – Phasen und enthalten min – destens einen optisch aktiven Oxiran – 2 – carbonsäureester.

Unter dem Begriff "Flüssigkristall-Phase" sind nematische cholesterische, orthogonal smektische oder geneigt ("tilted")-smektische, insbesondere $S_A^*$ -, $S_B^*$ - und $S_C^*$ -Phasen, zu verstehen. Die Flüssigkristallmischungen bestehen aus 2 bis 20, vorzugsweise 2 bis 15 Komponenten, darunter mindestens einer der erfindungsgemäß beanspruchten chiralen Verbindungen.

Die anderen Bestandteile werden vorzugsweise ausgewählt aus den bekannten Verbindungen mit nematischen, cholesterischen und/oder smektischen, z.B. $S_A$-Phasen, und/oder geneigt smektischen Phasen; dazu gehören beispielsweise Schiffsche Basen, Biphenyle, Terphenyle, Phenylcyclohexane, Cyclohexylbiphenyle, N-, S- oder O-haltige Heterocyclen (z.B. Pyrimidine), Zimtsäureester, Cholesterinester, verschieden überbrückte, terminalpolar mehrkernige Ester von p-Alkylbenzoesäuren. Im allgemeinen liegen die im Handel erhältlichen Flüssigkristallmischungen bereits vor der Zugabe der optisch aktiven Verbindung(en) als Gemische verschiedenster Komponenten vor, von denen mindestens eine mesogen ist, d.h. als Verbindung, in derivatisierter Form oder im Gemisch mit bestimmten Cokomponenten eine Flüssigkristallphase zeigt, die mindestens eine enantiotrope (Klärtemperatur > Schmelztemperatur) oder monotrope (Klärtemperatur < Schmelztemperatur) Mesophasenbildung erwärten läßt.
Insbesonder enthält die Flüssigkristall-Mischung neben mindestens einer der erfindungsgemäß beanspruchten optisch aktiven Verbindungen eine Esterverbindung mit $S_c$-Phase, z.B. einen Alkoxybenzoesäurephenylester, oder eine biaromatische Verbindung mit einem stickstoffhaltigen Heterocyclus, z.B. ein Alkylpyrimidinyl-alkoxy-benzol.

Von der oder den erfindungsgemäßen Verbindung(en) enthalten die Flüssigkristallmischungen im allgemeinen 0,05 bis 70 Gew.-%, insbesondere 0,1 bis 50 Gew.-%.
Die erfindungsgemäßen Verbindungen sind insbesondere als Dotierstoffe für geneigt-smektische Flüssigkristallphasen geeignet, da sie diese in ferroelektrische Flüssigkristall-Phasen umwandeln; die Werte für die spontane Polarisation ($P_s$) liegen bei 10 Mol-% Dotierung und bei 25 °C im Bereich von etwa 1-15 nC/cm$^2$ und im Bereich von etwa 10-150 nC/cm$^2$ linear extrapoliert auf die reine Verbindung, teilweise liegen die Werte für $P_s$ sogar noch höher.
Die Schaltzeiten der neuen Systeme liegen häufig unter 100 µs bei 10 Mol-% Dotierung, 25 °C und einer Schaltspannung von ± 10 V/µm. Besonders hervorstehend ist das Ergebnis des Substanzbeispiels 31, das die R,R-Konfiguration besitzt und die höchste spontane Polarisation, sowie die kürzeste Schaltzeit aufweist (siehe Tabelle 1, Anwendungsbeispiel A17). Die erfindungegemäßen Verbindungen können auch zur Erzielung des elektroklinen Effektes in orthogonalen smektischen Phasen ($S_A^*$, $S_B^*$, $S_E^*$) eingesetzt werden.

**Beispiel 1**

(2R,3S)-3-Nonyl-oxiran-2-carbonsäure-[4-(2-n-octylthiopyrimidin-5-yl)phenyl]-ester

[(I) mit $R^1$ = $H_{17}C_8S$, j = 1,

$-A^1$ = 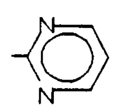 ,

k = l = m = null, n = 1,

$-A^3$ = 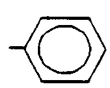 ,

X = O, $R^2$ = $C_9H_{19}$, $R^3$ = H]

Eine Lösung von 413 mg (2,0 mmol) Dicyclohexylcarbodiimid in 10 ml trochenem $CH_2Cl_2$ wird unter Rühren bei Raumtemperatur mit 429 mg (2mmol) (2R,3S)-3-Nonyloxiran-2-carbonsäure, 633 mg (2 mmol) 4-(2-n-Octylthiopyrimidin-5-yl)phenol sowie 20 mg 4-Dimethylaminopyridin versetzt. Nach 4 stündigem Rühren bei Raumtemperatur wird die Reaktionslösung im Vakuum eingeengt.
Nach chromatographischer Reinigung und Umkristallisation aus n-Hexan werden 120 mg (12 %) farblose Kristalle erhalten. Die Substanz zeigt die Flüssigkristallphasenfolge: X 73 $S_A$ 93,5 I.
Analog werden erhalten:

**Beispiel 2**

(2R,3S) – 3 – Pentyl – oxiran – 2 – carbonsäure – [4 – (2 – n – octylthiopyrimidin – 5 – yl)phenyl] – ester

[(I) mit $R^1$ = $H_{17}C_8S$, j = 1,

$-A^1$ = 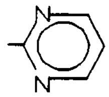 ,

k = l = m = null, n = 1,

$-A^3$ = 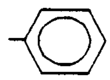 ,

X = O, $R^2$ = $C_5H_{11}$, $R^3$ = H]
Phasenfolge: X 61,4 $S_A$ 84,5 I

**Beispiel 3**

(2R,3S) – 3 – Pentyl – oxiran – 2 – carbonsäure – [4 – (2 – n – octyloxypyrimidin – 5 – yl)phenyl] – ester

[(I) mit $R^1$ = $H_{17}C_8O$, j = 1,

$-A^1$ = 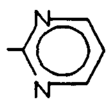 ,

k = l = m = null, n = 1,

$-A^3$ = 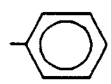 ,

X = O, $R^2$ = $C_5H_{11}$, $R^3$ = H]
Phasenfolge: X 47,5 $S_2$ 75,6 $S_1$ 115,8 I

**Beispiel 4**

(2R,3S) – 3 – Nonyl – oxiran – 2 – carbonsäure – [4 – (2 – n – octyloxypyrimidin – 5 – yl)phenyl] – ester

[(I) mit $R^1$ = $H_{17}C_8O$, j = 1,

$-A^1$ = 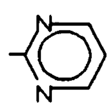 ,

k = l = m = null, n = 1,

$-A^3$ = 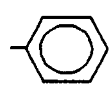 ,

X = O, $R^2$ = $C_9H_{19}$, $R^3$ = H]
Phasenfolge: X 55 $S_3$ 59 $S_2$ 93 $S_A$ 118 I

**Beispiel 5**

(2R,3S) – 3 – Nonyl – oxiran – 2 – carbonsäure – [4 – (2 – n – octyl – pyrimidin – 5 – yl)phenyl] – ester

[(I) mit $R^1$ = $H_{17}C_8$, j = 1,

$-A^1 =$ 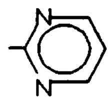 ,

k = 1 = m = null, n = 1,

$-A^3 =$ 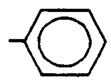 ,

X = O, $R^2$ = $C_9H_{19}$, $R^3$ = H]
Phasenfolge: X 48 $S_2$ 99 $S_A$ 105 I

**Beispiel 6**

(2R,3S) – 3 – Propyl – oxiran – 2 – carbonsäure – [4 – (2 – n – octylpyrimidin – 5 – yl)phenyl] – ester

[(I) mit $R^1$ = $H_{17}C_8$, j = 1,

$-A^1 =$ 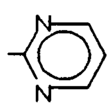 ,

k = l = m = null, n = 1,

$-A^3 =$ 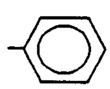 ,

X = O, $R^2$ = $C_3H_7$ , $R^3$ = H]
Phasenfolge: X 66 $S_A$ 86 I

**Beispiel 7**

(2R,3R) – 3 – Methyl – oxiran – 2 – carbonsäure[4 – (2 – n – octyl – pyrimidin – 5 – yl)phenyl] – ester

[(I) mit $R^1$ = $H_{17}C_8$ , j = 1,

$-A^1 =$ 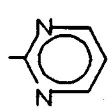 ,

k = l = m = null, n = 1,

$-A^3 =$ 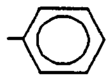 ,

X = O, $R^2$ = $CH_3$, $R^3$ = H]
Schmelzpunkt: 77 °C

**Beispiel 8**

(2R,3S)-3-Octyl-oxiran-2-carbonsäure[4-(5-n-nonyl-pyrimidin-2-yl)phenyl]-ester

[(I) mit $R^1$ = $H_{19}C_9$ , j = 1,

$-A^1 =$ 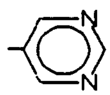 ,

k = 1 = m = null, n = 1,

$-A^3 =$ 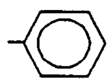 ,

X = O, $R^2$ = $C_8H_{17}$, $R^3$ = H]
Phasenfolge: X 53 $S_A$ 63 N* 64 I

**Beispiel 9**

(2R,3S)-3-Propyl-oxiran-2-carbonsäure-[4-(5-n-octylpyrimidin-2-yl)phenyl]-ester

[(I) mit $R^1$ = $H_{17}C_8$ , j = 1,

$-A^1 =$ 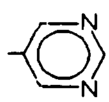 ,

k = l = m = null, n = 1,

$-A^3 =$ 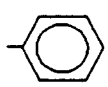 ,

X = O, $R^2$ = $C_3H_7$ , $R^3$ = H]
Schmelzpunkt: 62 °C

**Beispiel 10**

(2R,3R) – 3 – Methyl – oxiran – 2 – carbonsäure[4 – (5 – n – octyl – pyrimidin – 2 – yl)phenyl] – ester

[(I) mit $R^1 = H_{17}C_8$ , j = 1,

$-A^1 =$ 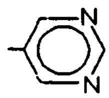 ,

k = l = m = null, n = 1,

$-A^3 =$ 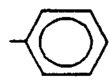 ,

X = O, $R^2 = C_3$ , $R^3 = H$]
Schmelzpunkt: 67 °C

**Beispiel 11**

(2R,3S) – 3 – [trans – 4 – (trans – 4 – heptylcyclohexyl)cyclohexyl] – oxiran – 2 – carbonsäure – (4 – decyloxy – phenyl) – ester

[(I) mit $R^1 = H_{21}C_{10}O$, j = k = l = m = null, n = 1,

$-A^3 =$ 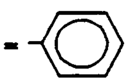 ,

X = O,

$R^2 =$ 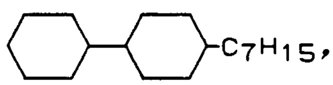

$R^3 = H$]
Phasenfolge: X 67,2 $S_B$ 125,9 $S_A$ 164,4 I

**Beispiel 12**

(2R,3S) – 3 – [trans – 4 – (trans – 4′ – Heptylcyclohexyl)cyclohexyl] – oxiran – 2 – carbonsäure – (4 – dodecyloxy – phenyl) – ester

[(I) mit $R^1 = H_{25}C_{12}O$, j = k = l = m = null, n = 1,

$-A^3 =$ 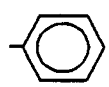 ,

X = O,

$R^2$ = 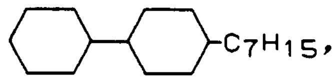

$R^3$ = H]

Phasenfolge: X 79 $S_1$ 156 I

**Beispiel 13**

(2R,3S) – 3 – (trans – 4 – Pentyl – cyclohexyl)oxiran – 2 – carbonsäure – (4 – dodecyloxy – phenyl) – ester

[(I) mit $R^1$ = $H_{25}C_{12}O$, j = k = l = m = null, n = 1,

$-A^3$ = 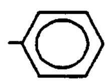 ,

X = O,

$R^2$ = 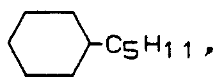

$R^3$ = H]

Schmelzpunkt: 69 °C

**Beispiel 14**

(2R,3S) – 3 – Octyl – oxiran – 2 – carbonsäure – (4′ – octyloxy – biphenyl – 4 – yl) – ester

[(I) mit $R^1$ = $H_{17}C_8O$, j = 1,

$-A^1$ = 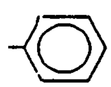 ,

k = l = m = null, n = 1,

$-A^3$ = 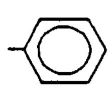 ,

X = O, $R^2$ = $C_8H_{17}$, $R^3$ = H]

Phasenfolge: X 117,5 $S_2$ 118 $S_A$ 119 I

**Beispiel 15**

(2R,3S) – 3 – Propyl – oxiran – 2 – carbonsäure – [4 – (4 – decyloxybenzoyloxy)phenyl] – ester

[(I) mit $R^1 = H_{21}C_{10}O$, j = 1,

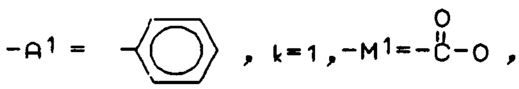

l = m = null, n = 1,

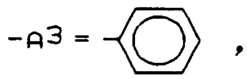

X = O, $R^2 = C_3H_7$, $R^3$ = H]
Phasenfolge: X 82,5 N* 103,5 I

**Beispiel 16**

(2R,3S) – 3 – Octyl – oxiran – 2 – carbonsäure – [4 – (4 – decyloxybenzoyloxy)biphenyl – 4′ – yl] – ester

[(I) mit $R^1 = H_{21}C_{10}O$, j = 1,

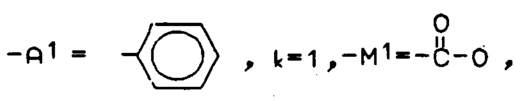

l = m = null, n = 2,

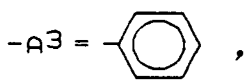

X = O, $R^2 = C_8H_{17}$, $R^3$ = H]
Phasenfolge: X 117 $S_2$ 122 $S_C^*$ 186 N* 200 I

**Beispiel 17**

(2R,3S) – 3 – Nonyl – oxiran – 2 – carbonsäure – [4 – (4′ – octyloxybiphenyl – 4 – carbonyloxy)phenyl] – ester

[(I) mit $R^1 = H_{17}C_8O$, j = 2,

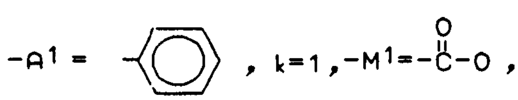

l = m = null, n = 1,

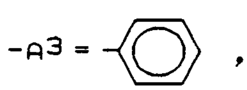

$X = O$, $R^2 = C_9H_{19}$, $R^3 = H$]
Schmelzpunkt: 139 °C

**Beispiel 18**

(2R,3S) – 3 – Nonyl – oxiran – 2 – carbonsäure – [4 – (4 – (4 – octyloxybenzoyloxy)benzoyloxy)phenyl] – ester

[(I) mit $R^1 = H_{17}C_8O$, $j = 1$,

$-A^1 =$ 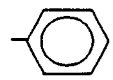 , $k = 1$, $-M^1 =$ 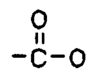 ,

$l = 1$,

$-A^2 =$ 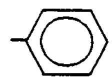 ,

$m = 1$,

$-M^2 =$ 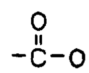 ,

$n = 1$,

$-A^3 =$ 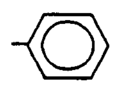

$X = O$, $R^2 = C_9H_{19}$, $R^3 = H$]
Phasenfolge: X 118 $S_C^*$ 167 $S_A^*$ 177 N* 207 I

**Beispiel 19**

(2R,3S) – 3 – Nonyl – oxiran – 2 – carbonsäure – [4 – (4 – octyloxybenzyloxy)phenyl] – ester

[(I) mit $R^1 = H_{17}C_8O$, $j = 1$,

$-A^1 =$ 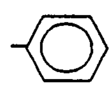 ,

$k = 1$, $-M^1 = -CH_2O$, $l = m =$ null, $n = 1$,

$-A^3 =$ 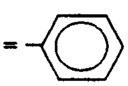 ,

$X = O$, $R^2 = C_9H_{19}$, $R^3 = H$]
Schmelzpunkt: 101 °C

**Beispiel 20**

(2R,3S) – 3 – Nonyl – oxiran – 2 – carbonsäure – [4 – (4′ – octyl – biphenyl – 4 – methylenoxy)phenyl] – ester

[(I) mit $R^1$ = $H_{17}C_8$, j = 2,

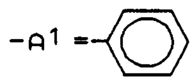,

k = 1, $-M^1 = -CH_2O$, l = m = null, n = 1,

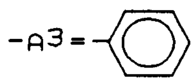,

X = O, $R^2$ = $C_9H_{19}$, $R^3$ = H]
Schmelzpunkt: 164 °C

**Beispiel 21**

Bis[(2R,3S – 3 – Nonyl – oxiran – 2 – carbonsäure] – 4,4′ – biphenyl – ester

[(I) mit $R^1$ = (2R,3S) – 3 – Nonyl – oxiron – 2 – carbonyloxy, $R^2$ = $C_9H_{19}$, $R^3$ = H, j = k = l = m = null, n = 2,

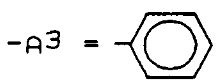,

X = O]
Schmelzpunkt: 158 °C

**Beispiel 22**

(2R,3S) – 3 – Nonyl – oxiran – 2 – carbonsäure – [ – (4 – decyloxybenzoyloxy)phenyl] – ester

[(I) mit $R^1$ = $H_{21}C_{10}O$, j = 1,

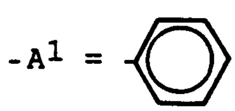,

k = l,

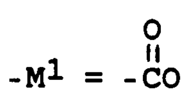,

l = m = null, n = 1,

$-A^3$ = 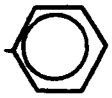 ,

X = O, $R^3$ = $C_9H_{19}$, $R^3$ = H]
Phasenfolge: X 91 $S_C$ 102 N* 105 I

**Beispiel 23**

(2R,3R) – 3 – Propyl – oxiran – 2 – carbonsäure – [4 – (2 – n – octylpyrimidin – 5 – yl)phenyl] – ester

[(I) mit $R^1$ = $H_{17}C_8$, j = 1,

$-A^1$ = 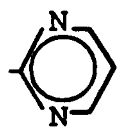 ,

k = l = m = null, n = 1,

$-A^3$ = 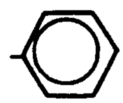 ,

X = O, $R^2$ = $C_3H_7$, $R^3$ = H]
Schmelzpunkt: 83 °C

**Beispiel 24**

(2R,3R) – 3 – Propyl – oxiran – 2 – carbonsäure – [ – (5 – n – octyl – pyrimidin – 2 – yl)phenyl] – ester

[(I) mit $R^1$ = $H_{17}C_8$, j = 1,

$-A^1$ = 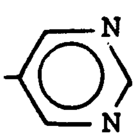 ,

k = l = m = null, n = 1,

$-A^3$ = 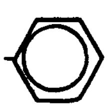 ,

X = O, $R^2$ = $C_3H_7$, $R^3$ = H]
Schmelzpunkt: 40 °C

**Beispiel 25**

(2R,3R) – 3 – Methyl – oxiran – 2 – carbonsäure – [4 – (2 – n – octylthiopyrimidin – 5 – yl)phenyl] – ester

[(I) mit $R^1 = H_{17}C_8S$, j = 1,

$-A^1 =$ 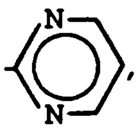,

k = l = m = null, n = 1,

$-A^3 =$ 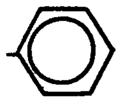,

X = O, $R^2 = CH_3$, $R^3 = H$]
Schmelzpunkt: 67 °C

**Beispiel 26**

(2R,3R) – 3 – Methyl – oxiran – 2 – carbonsäure – [4 – (2 – n – octyloxypyrimidin – 5 – yl)phenyl] – ester

[(I) mit $R^1 = H_{17}C_8O$, j = 1,

$-A^1 =$ 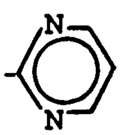,

k = l = m = null, n = 1,

$A^3 =$ 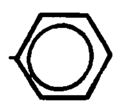,

X = O, $R^2 = CH_3$, $R^3 = H$]
Schmelzpunkt: 97 °C

**Beispiel 27**

(2R,3R) – 3 – Methyl – oxiran – 2 – carbonsäure – [4 – (2 – ((S) – 7 – methylnonyloxy) – pyrimidin – 5 – yl) – phenyl] – ester

[(I) mit

$R^1 = H_5C_2\underset{*}{C}H(CH_3)(-CH_2)_6O$, j = 1, $-A^1 =$ 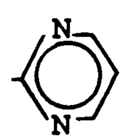,

k = l = m = null, n = 1,

$-A^3 =$ 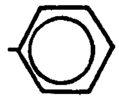 ,

X = O, $R^2$ = $CH_3$, $R^3$ = H]
Schmelzpunkt: 87 °C

**Beispiel 28**

(2R,3R) – 3 – Propyl – oxiran – 2 – carbonsäure – [4 – (2 – ((S) – 7 – methylnonyloxy) – pyrimidin – 5 – yl) – phenyl] – ester

[(I) mit

$R^1 = H_5C_2\overset{}{\underset{*}{C}}H(CH_3)(-CH_2)_6O$, j = 1, $-A^1 =$ 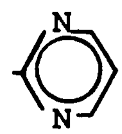 ,

k = l = m = null, n = 1,

$-A^3 =$ 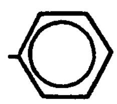 ,

X = O, $R^2$ = $C_3H_7$, $R^3$ = H]
Schmelzpunkt: 45 °C

**Beispiel 29**

(2R,3R) – 3 – Propyl – oxiran – 2 – carbonsäure – [4 – (2 – octyloxypyrimidin – 5 – yl)]phenyl – ester

[(I) mit $R^1$ = $H_{17}C_8O$, j = 1,

$-A^1 =$ 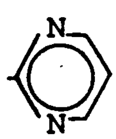 ,

k = l = m = null, n = 1,

$-A^3 =$ 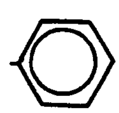 ,

X = O, $R^2$ = $C_3H_7$, $R^3$ = H]
Schmelzpunkt: 62 °C

**Beispiel 30**

(2R,3R) – 3 – Propyl – oxiran – 2 – carbonsäure – [4 – (5 – octyloxypyrimidin – 2 – yl)]phenyl – ester

[(I) mit $R^1 = H_{17}C_8O$, j = 1,

$-A^1 =$ 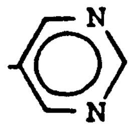 ,

k = l = m = null, n = 1,

$A^3 =$ 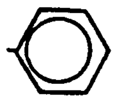 ,

X = O, $R^2 = C_3H_7$, $R^3$ = H]
Schmelzpunkt: 67 °C

**Beispiel 31**

(2R,3R) – 3 – Propyl – oxiran – 2 – carbonsäure – [2 – (4 – octyloxy – phenyl) – pyrimidin – 5 – yl] – ester

[(I) mit $R^1 = H_{17}C_8O$, j = 1,

$-A^1 =$ 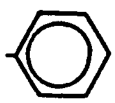 ,

k = l = m = null, n = 1,

$-A^3 =$ 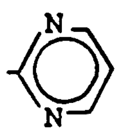 ,

X = O, $R^2 = C_3H_7$, $R^3$ = H]
Schmelzpunkt: 83 °C

**Beispiel 32**

(2R,3R) – 3 – Propyl – oxiran – 2 – carbonsäure – [4 – (4 – octyloxybenzoyloxy) – biphenyl – 4' – yl] – ester

[(I) mit $R^1 = H_{17}C_8O$, j = k = 1,

$A^1 =$ 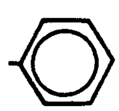 ,

$-M^1 = -CO-O$, l = m = 0, n = 2,

$A^3 =$ 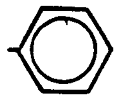 ,

X = O, $R^2 = C_3H_7$, $R^3$ = H]
Phasenfolge: X ($S_A^*$ 121 N* 123) 131 I

**Beispiel 33**

(2R,3R)-3-Propyl-oxiran-2-carbonsäure-[4-(2-(10-undecen-1-yl)oxy-pyrimidin-5-yl)-phenyl]-ester

[(I) mit $R^1 = H_2C = CH(-CH_2)_9O$, j = 1,

$-A^1 =$ 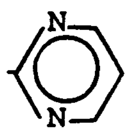 ,

k = l = m = null, n = 1,

$-A^3 =$ 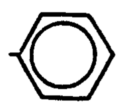 ,

X = O, $R^2 = C_3H_7$, $R^3$ = H]
Schmelzpunkt: 61 °C

**Beispiel 34**

(2R,3R)-3-Propyl-oxiran-2-carbonsäure-[4-(2-(1,1-H-perfluoroctyl)oxy-pyrimidin-5-yl)-phenyl]-ester

[(I) mit $R^1 = F_{15}C_7CH_2O$, j = 1,

$-A^1 =$ 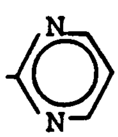 ,

k = l = m = null, n = 1,

$-A^3 =$ 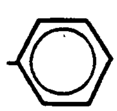 ,

X = O, $R^2 = C_3H_7$, $R^3$ = H]
Schmelzpunkt: 99 °C

**Beispiel 35**

(2R,3R) – 3 – Propyl – oxiran – 2 – carbonsäure – [4 – (5 – octyl – 1,3 – dioxan – 2 – yl)phenyl] – ester

[(I) mit $R^1 = H_{17}C_8$, j = 1,

$-A^1 =$ 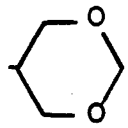 ,

k = l = m = null, n = 1,

$-A^3 =$ 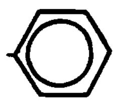 ,

X = O, $R^2 = C_3H_7$, $R^3 = H$]
Schmelzpunkt: 59 °C

**Beispiel 36**

(2R,3R) – 3 – Propyl – oxiran – 2 – carbonsäure – [4 – (2 – nonyloxypyrimidin – 5 – yl)phenyl] – ester

[(I) mit $R^1 = H_{19}C_9O$, j = 1,

$-A^1 =$ 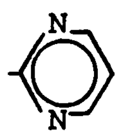 ,

k = l = m = null, n = 1,

$-A^3 =$ 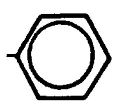 ,

X = O, $R^2 = C_3H_7$, $R^3 = H$]
Schmelzpunkt: 68 °C

**Beispiel 37**

(2R,3R) – 3 – Propyl – oxiran – 2 – carbonsäure – [4 – (2 – decyloxypyrimidin – 5 – yl)phenyl] – ester

[(I) mit $R^1 = H_{21}C_{10}O$, j = 1,

$-A^1 =$ 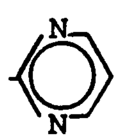 ,

k = l = m = null, n = 1,

$-A^3 =$ 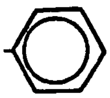 ,

X = O, $R^2 = C_3H_7$, $R^3$ = H]
Schmelzpunkt: 70 °C

**Beispiel 38**

(2R,3R)-3-Propyl-oxiran-2-carbonsäure-[4-(5-octyl-pyridin-2-yl)phenyl]-ester

[(I) mit $R^1 = H_{17}C_8$, j = 1,

$-A^1 =$ 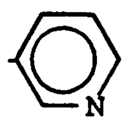 ,

k = 1 = m = null, n = 1,

$-A^3 =$ 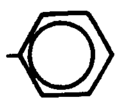 ,

X = O, $R^2$ $C_3H_7$, $R^3$ = H]
Schmelzpunkt: 53 °C

**Beispiel 39**

(2R,3R)-3-Propyl-oxiran-2-carbonsäure-[2-(4-pentyloxycarbonyl-phenyl)-pyrimidin-5-yl]-ester

[(I) mit $R^1 = H_{11}C_5-CO-O$, j = 1,

$-A^1 =$ 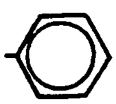 ,

k = l = m = null, n = 1,

$-A^3 =$ 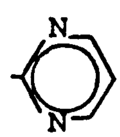 ,

X = O, $R^2 = C_3H_7$, $R^3$ = H]
Schmelzpunkt: 82 °C

**Beispiel 40**

(2R,3R) – 3 – Propyl – oxiran – 2 – carbonsäure – [4 – (4 – trans – pentylcyclohexyl)phenyl] – ester

[(I) mit $R^1$ = $H_7C_3$, j = 1,

$-A^1$ = 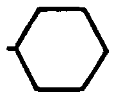 ,

k = l = m = null, n = 1,

$-A^3$ = 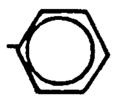 ,

X = O, $R^2$ = $C_3H_7$, $R^3$ = H]
Schmelzpunkt: 46 °C

**Beispiel 41**

(2R,3R) – 3 – Propyl – oxiran – 2 – carbonsäure – [4 – (2 – (4 – hexyloxyphenyl)pyrimidin – 5 – yl)phenyl] – ester

[(I) mit $R^1$ = $H_{13}C_6O$, j = l = n = 1, k = m = null,

$-A^1$ = 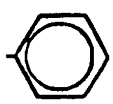 -, $-A^2$ = 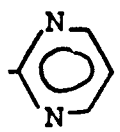 - , $-A^3$ = 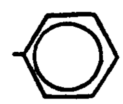 ,

X = O, $R^2$ = $C_3H_7$, $R^3$ = H]
Phasenfolge: X 121 $S_C^*$ 151 I

**Beispiel 42**

(2R,3R) – 3 – Propyl – oxiran – 2 – carbonsäure – [2 – (4 – (5 – oxo – hexyl) – oxy – phenyl)pyrimidin – 5 – yl] – ester

[(I) mit $R^1$ = $H_3C-CO(-CH_2)_4O$, j = 1,

$-A^1$ = 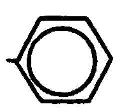 ,

k = l = m = null, n = 1,

$-A^3$ = 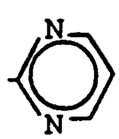 ,

X = O, $R^2$ = $C_3H_7$, $R^3$ = H]
Schmelzpunkt: 84 °C

**Beispiel 43**

(2R,3R) – 3 – Propyl – oxiran – 2 – carbonsäure – [4 – (5 – octyl – 1,3 – dithian – 2 – yl)phenyl] – ester

[(I) mit $R^1 = H_{17}C_8$, j = 1,

$-A^1 =$ 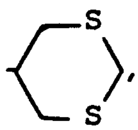,

k = l = m = null, n = 1,

$-A^3 =$ 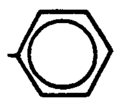,

X = O, $R^2 = C_3H_7$, $R^3 = H$]
Schmelzpunkt: 102 °C

**Beispiel 44**

(2R,3R) – 3 – Propyl – oxiran – 2 – carbonsäure – [2 – (4 – (4 – transpropylcyclohexyl)carbonyloxy – phenyl) – pyrimidin – 5 – yl] – ester

[(I) mit $R^1 = H_7C_3$, j = k = l = n = 1, m = null,

$-A^1 =$ 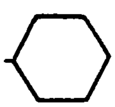, $M^1 = -CO-O$, $-A^2 =$ 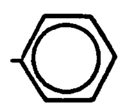, $-A^3 =$ 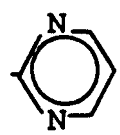,

X = O, $R^2 = C_3H_7$, $R^3 = H$]
Schmelzpunkt: 141 °C

**Beispiel 45**

(2R,3R) – 3 – Methyl – oxiran – 2 – carbonsäure – [4 – (2 – ((S) – 8 – methyldecyl) – pyrimidin – 5 – yl)phenyl] – ester

[(I) mit

$R^1 = H_5C_2\underset{*}{C}H(CH_3)(-CH_2)_7$, j = 1, $-A^1 =$ 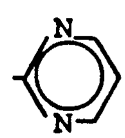,

k = l = m = null, n = 1,

$-A^3 =$ 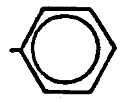 ,

X = O, $R^2 = CH_3$ $R_3$ = H]
Schmelzpunkt: 73 °C

**Anwendungsbeispiel A1 bis A20**

Zur Überprüfung der Wirksamkeit der vorstehend beschriebenen Verbindungen als ferroelektrische Dotierstoffe in Flüssigkristall-System mit geneigten smektischen Phasen werden diese in Konzentrationen von jeweils 10 Mol-% mit dem Racemat der Verbindung (A)

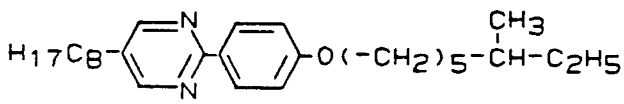 (A)

Phasenfolge: K 14,9 $S_c$ 49,8 $S_A$ 59,2 I (5 °C)
4-(5-Octyl-pyrimidin-2-yl)-1-(6-methyl-octyl-1-oxy)benzol bzw. der Verbindung (B)

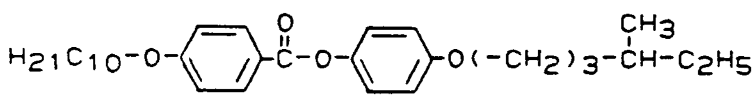 (B)

Phasenfolge: K 17 $S_G$ 32,7 $S_c$ 70,4 $S_A$ 73,3 I (- 3 °C)
4-(4-Decyloxy-phenyl-1-carbonyloxy)-1-(4-methyl-hexyl-oxy)benzol bzw. einer nicht-chiralen Grundmischung (C)
mit der Zusammensetzung

60 % 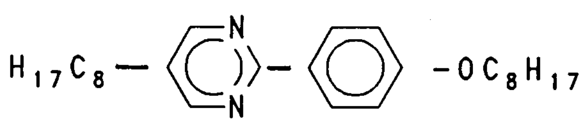

40 % 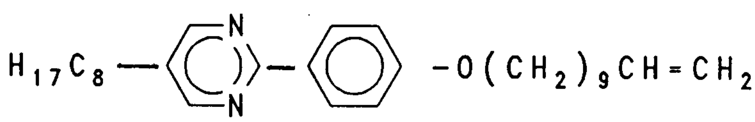

un der Phasenfolge

K 13 $S_C$ 51 $S_A$ 62 N 67 I (C)

bzw. einer weiteren nicht chiralen Grundmischung (D) mit der Zusammensetzung

15 % 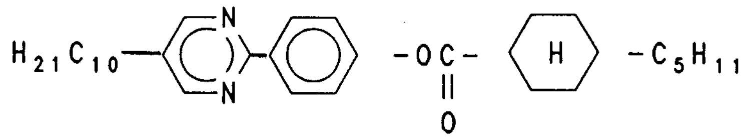

21,24 % 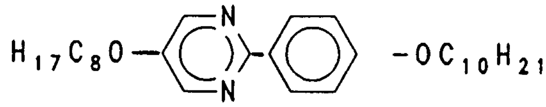

26,72 % 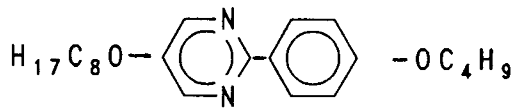

25,33 % 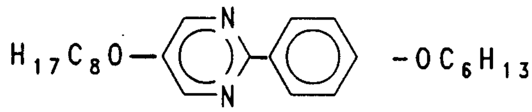

11,69 % 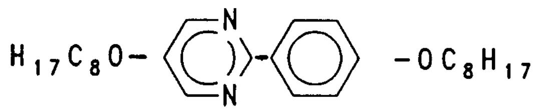

und der Phasenfolge

K 12,5 $S_C$ 83 $S_A$ 95 N 100 I (D)

gemischt.

Es wurden jeweils die Werte für die spontane Polarisation ($P_s$ in $nC \cdot cm^{-2}$), für die Schaltzeit $\tau$ (in $\mu s$) und für den optischen Neigungswinkel der $S_c$-Phase $\theta$ (in °) der Mischung bestimmt. Die $P_s$-Werte werden nach der Methode von H. Diamant et al. (Rev. Sci. Instr., 28, 30, 1957) gemessen, wobei eine spezielle Meßzelle [Skarp et al. in Ferroelectric Letters Vol. 06, 67 (1986)] verwendet wird, in der auch die $\tau$- und $\theta$-Werte bestimmt werden. Bei einer Zellenschichtdicke von ca. 2 $\mu m$ wird durch Scherung eine einheitliche planare Orientierung der Flüssigkristalle in der $S_c$-Phase erreicht [SSFLC-Technik, Clark et al., Appl. Phys. Lett. 36, 899 (1980)]. Zur Bestimmung von $\tau$ und $\theta$ wird die Meßzelle auf dem Drehtisch eines Polarisationsmikroskops zwischen gekreuztem Analysator und Polarisator befestigt. Durch Drehen der Meßzelle von maximalen zu minimalen Lichtdurchgang wird der optische Neigungswinkel bzw. Schaltwinkel 2 $\theta$ bestimmt. Mit Hilfe einer Photodiode erfolgt die Bestimmung der Schaltzeit $\tau$ indem die Anstiegszeit des Lichtsignals von 10 auf 90 % Signalhöhe gemessen wird. Die Schaltspannung beträgt ± 10 V/$\mu m$. Neben den Werten für $P_s$, $\tau$, $2\theta$ ist der $S_c$-Bereich der jeweiligen Mischung angegeben; die Werte in Klammern geben dabei die unterkühlbare untere Temperaturgrenze des $S_c$-Bereichs an.

Die Tabelle 1 faßt die Ergebnisse zusammen.

**Tabelle 1**

| (Substanz-) beispiel | Anwendungs- beispiel | Wirt | Temp. °C | $S_C^*$-Bereich in der Mischung in °C | $P_s$ nC/cm² | τ µs | 2θ Grad |
|---|---|---|---|---|---|---|---|
| 11 | A1 | B | 25 | 34-65 | 6,4 | 76 | 45 |
| 12 | A2 | B | 25 | [35]-65 | 4 | 130 | 44 |
| 13 | A3 | A | 40 | [5]-40 | 2 | 200 | 28 |
| 14 | A4 | A | 40 | 8-50 | 1,3 | 180 | 44 |
| 8 | A5 | A | 40 | 5-46 | 6 | 75 | 38 |
| 15 | A6 | B | 25 | [30]-58 | 11 | 35 | 58 |
| 3 | A7 | C | 25 | 7[5]-40 | 3 | 55 | 35 |
| 2 | A8 | C | 25 | 32-46 | 3,2 | 290 | 38 |
| 7 (R,R) | A9 | C | 25 | 28[20]-44 | 15 | 19 | 36 |
| 24 | A10 | D | 25 | 10-68,5 [-8] | 55,8 | 14/28 | 50/30 |
| 25 | A11 | D | 25 | 10-73,5 | 18,2 | 20/48 | 50/22 |
| 26 | A12 | D | 25 | 14-78 [-5] | 46,4 | 21/57 | 55/13 |
| 27 | A13 | D | 25 | 13-78 | 47 | 26/70 | 52/15 |
| 28 | A14 | D | 25 | 8-77,5 [-6,5] | 63,9 | 21/55 | 59/14 |
| 29 | A15 | D | 25 | 9-77,5 [-6] | 62 | 25/62 | 60/21 |
| 30 | A16 | D | 25 | 9-73 [-6] | 45,4 | 25/50 | 52/24 |
| 31 | A17 | D | 40 | 50-73 [+21] | 78,3 | 6,6/ 17,8 | 62/18 |
| 36 | A18 | D | 25 | 10-79 [-5] | 63 | 21,5/ 55 | 57/24 |
| 37 | A19 | D | 25 | 10-79,5 [-4] | 70,3 | 15,6/ 45,4 | 58/21 |
| 45 | A20 | D | 25 | 10-71 [-7] | 45,4 | 19/46 | 52/16 |

[] Die mit einer Klammer gekennzeichneten Temperaturen sind Unterkühlungstemperaturen

**Anwendungsbeispiels A21 bis A30**

In die nicht-chirale Flüssigkristallmischung (C) mit der Phasenfolge I → N → $S_A$ → $S_C$ wurde jeweils eine der erfindungsgemäßen Verbindungen zugemischt und deren Verdrillungsvermögen (Erzeugung einer Helix) in der nematischen und der $S_C^*$ -Phase untersucht. Die Bestimmung der Ganghöhe der induzierten Helix erfolgte, wie bei P. Kassubek et al., Mol. Cryst. Liq. Cryst., Vol. 8, Seite 305 bis 314, 1969 beschrieben, in einer Keilzelle mit Orientierungsschicht durch Ausmessen der Versetzungslinien unter dem Polarisationsmikroskop. Die Bestimmung der Ganghöhe in der $S_C^*$ -Phase erfolgte anhand des Beugungsmusters eines He-Ne-Laserstrahls (K. Kondo et al. Jpn. J. Appl. Phys. 21, 224 (1982)) sowie durch Vermessung der Versetzungsstreifen im Polarisationsmikroskop. Tabelle 2 faßt die Ergebnisse zusammen.

Besonders auffällig ist das enorme Verdrillungsvermögen der Substanz des Beispiels 10 (Anwendungsbeispiel A21), die daher sowohl in der N*- als auch in der $S_C^*$ -Phase hervorragend zur pitch-Kompensation in Flüssigkristallmischungen geeignet ist.

Tabelle 2

| (Substanz-) beispiel | Anwendungs- beispiel | Temperatur °C | HTP[1)] 1/µm | Phase |
|---|---|---|---|---|
| 10 | A21 | 62 | 7,5 | N* |
| 3 | A22 | 25 | 15,1 | $S_C^*$ |
| | | 60 | 11,4 | N* |

**Fortsetzung der Tabelle 2**

| (Substanz-) beispiel | Anwendungs-beispiel | Temperatur °C | HTP[1)] 1/µm | Phase |
|---|---|---|---|---|
| 7 | A23 | 25 | 15,6 | $S_C^*$ |
| 26 | A24 | 63 | 8,3 | N* |
| 27 | A25 | 62,5 | 9,5 | N* |
| 28 | A26 | 91,5 | 4,6 | N* |
| 29 | A27 | 91,5 | 5,8 | N* |
| 30 | A28 | 94 | 4,5 | N* |
| 31 | A29 | 89 | 9,1 | N* |
| 36 | A30 | 92 | 5,8 | N* |

1) HTP (helical twisting power) = $(x \cdot p)^{-1}$
x = Molenbruch des Dotierstoffes in der Basismischung C
p = pitch

**Anwendungsbeispiel A31**

Die Verbindungen der Formel (I) induzieren in orthogonalen smektischen Phasen ($S_A$, $S_B$, $S_E$) den elektroklinen Effekt, dessen Größe durch den Differentialkoeffizienten ($d\theta/dE$) angegeben wird. $\theta$ ist der vom elektrischen Feld E induzierte Neigungswinkel. Die Messung dieser Größe erfolgte in der $S_A^*$ – Phase, jedoch in derselben "bookshelf" – Anordnung und in derselben Zelle, die auch für die Polarisationsmessungen verwendet wurde (siehe Anwendungsbeispiel A1 bis A20). Der durch das elektrische Feld induzierte Neigungswinkel wird im Polarisationsmikroskop bei gekreuzten Polarisatoren gemessen, indem man die Dunkelstellungen auffindet, die zugehörigen Winkel am Drehtisch abliest und von diesen die feldfrei gemessenen Winkel abzieht. Zur Überprüfung der Wirksamkeit der Verbindungen (I) wurde der elektrokline Koeffizient der im Anwendungsbeispiel A9 definierten Mischung bei einer Temperatur von 48 °C bestimmt. Wir erhielten ($d\theta/dE$) = $2{,}5 \cdot 10^{-9}$ rad m/V.

**Patentansprüche**

1. Verwendung von optisch aktiven Oxiran – 2 – carbonsäureestern als Dotierstoffe in Flüssigkristallmischungen.

2. Verwendung nach Anspruch 1, dadurch gekennzeichnet, daß Oxiran – 2 – carbonsäureester der allgemeinen Formel I verwendet werden

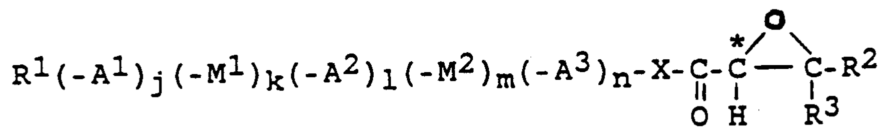 (I)

in der die Symbole und Indices folgende Bedeutung haben:

$R^1$

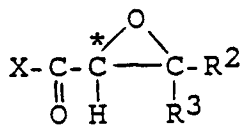

oder ein geradkettiger oder verzweigter Alkylrest mit 1 bis 16 C-Atomen oder ein geradkettiger oder verzweigter Alkenylrest mit 3 bis 16 C-Atomen, wobei diese Reste selbst asymmetrische C-Atome enthalten können, eine oder mehrere nicht benachbarte $-CH_2-$Gruppen durch $-O-$, $-S-$, $-CO-$, $-O-CO-$ und/oder $-CO-O-$ ersetzt sein können, und wobei ein oder mehrere H durch F, Cl, Br ersetzt sein können

$R^2$ H oder Alkyl mit $C_1-C_{16}$, oder Cyclohexyl oder Bicyclohexyl, die auch jeweils in Position 4 bzw. 4' durch eine Alkylkette mit 1 bis 16 C-Atomen substituiert sein können,

$R^3$ H

j und l null, 1 oder 2

k und m null oder 1

n null, 1 oder 2

mit folgender Maßgabe: wenn j und/oder l = null sind, ist k = null; wenn n = null ist, ist m = null; die Summe j + l + n ist minimal 1 und maximal 3,

$-A^1$, $-A^2$

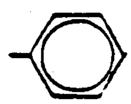 , 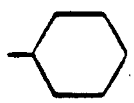 , 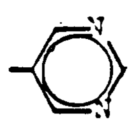 , 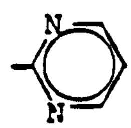 ,

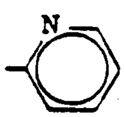 , 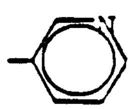 , 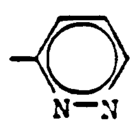 , 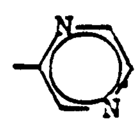 ,

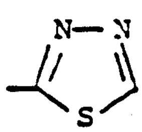 , 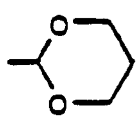 , 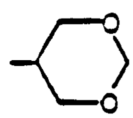 , 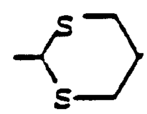 ,

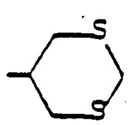 , 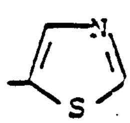 , 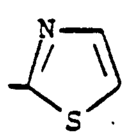 , 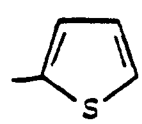 ,

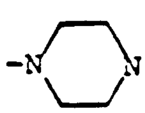 ,

$-A^3$

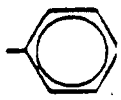 , 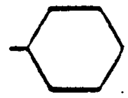 , 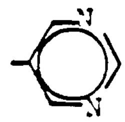 , 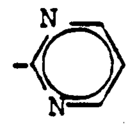 ,

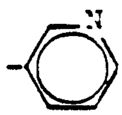 , 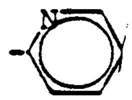 , 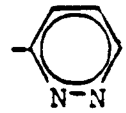

$-M^1$, $-M^2$ $-CO-O$, $-O-CO$, $-CH_2O$ , $-OCH_2$

X O.

**3.** Verwendung nach Anspruch 2, dadurch gekennzeichnet, daß Oxiran – 2 – carbonsäureester der allgemeinen Formel (IV) verwendet werden

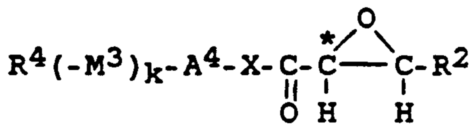 (IV)

worin bedeuten:

$R^4$ einen geradkettigen oder verzweigten Alkyl – oder Alkenylrest mit 6 bis 12 C – Atomen, der ein asymmetrisches C – Atom enthalten kann;

$-M^3$ $-O$, $-S$, $-O-CO$ oder $-CO$

$-A^4$

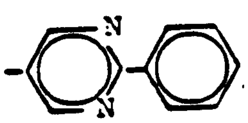 , 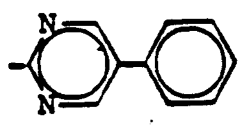 , 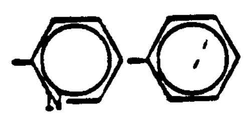 ,

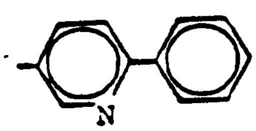 , 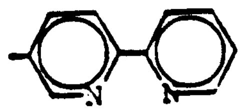 , 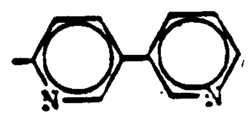 ,

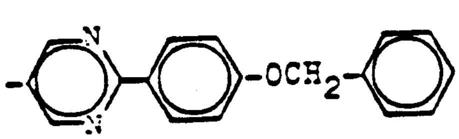 , 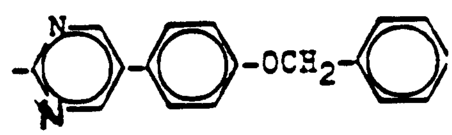 ,

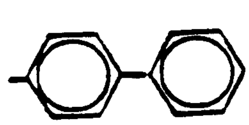 , 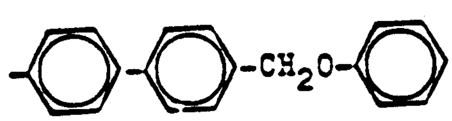 ,

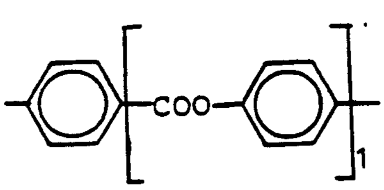 1 oder 2

**4.** Optisch aktive Oxiran – 2 – carbonsäureester der allgemeinen Formel (I) nach Anspruch 2.

**5.** Optisch aktive Oxiran – 2 – carbonsäureester der allgemeinen Formel (IV) nach Anspruch 3.

**6.** Flussigkristallmischunt, gekennzeichnet durch einen Gehalt an mindestens einem optisch aktiven Oxiran – 2 – carbonsäureester.

**7.** Flüssigkristallmischung, gekennzeichnet durch einen Gehalt an mindestens einem optisch aktiven Oxiran – 2 – carbonsäureester der allgemeinen Formel (I) nach Anspruch 2.

**8.** Flüssigkristallmischung, gekennzeichnet durch einen Gehalt an mindestens einem optisch aktiven Oxiran – 2 – carbonsäureester der allgemeinen Formel (IV) nach Anspruch 3.

**9.** Elektrooptisches Schalt – oder Anzeigeelement enthaltend eine Flüssigkristallmischung nach Anspruch 6.

**10.** Verfahren zur Herstellung von optisch aktiven Oxiran – 2 – carbonsäureestern der allgemeinen Formel (I) nach Anspruch 2, dadurch gekennzeichnet, daß man mesogene Phenole der allgemeinen Formel (II) mit Derivaten der Oxiran – 2 – carbonsäure der allgemeinen Formel (III) umsetzt, wobei Y eine OH – Gruppe oder Halogen bedeutet:

$R^1(-A^1)_j(-M^1)_k(-A^2)_l(-M^2)_m(-A^3)_n-X-H$ (II)

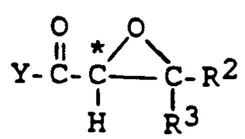

(III)

## Claims

**1.** The use of optically active oxirane – 2 – carboxylic acid esters as dopants in liquid – crystal mixtures.

**2.** The use as claimed in claim 1, wherein oxirane – 2 – carboxylic acid esters of the formula 1 are used

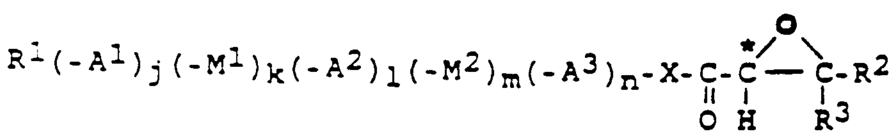

(I)

in which the symbols and indices have the following meaning:

$R^1$ =

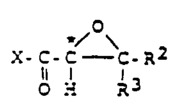

or a straight – chain or branched alkyl radical containing 1 to 16 carbon atoms or a straight – chain or branched alkenyl radical containing 3 to 16 carbon atoms, it being possible for said radicals themselves to contain asymmetric carbon atoms, it being possible for one or more nonadjacent $CH_2$ groups to be replaced by – O –, – S –, – CO –, – O – CO – and/or – CO – O –, and it being possible for one or more hydrogen atoms to be replaced by F, Cl or Br,

$R^2$ = H or alkyl containing 1 to 16 carbon atoms or cyclohexyl or bicyclohexyl which may also be substituted in each case in position 4 or 4' by an alkyl chain containing 1 to 16 carbon atoms,

$R^3$ = H
j and l = zero, 1 or 2,
k and m = zero or 1,
n = zero, 1 or 2,

with the following proviso: if j and/or l = zero, k = zero; if n = zero, m = zero; the sum of j + l + n is minimally = 1 and is maximally = 3,

$-A^1$, $-A^2$ =

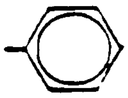 , 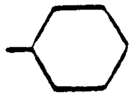 , 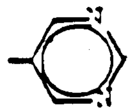 , 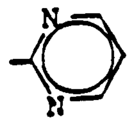 ,

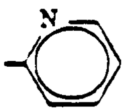 , 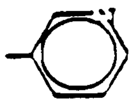 , 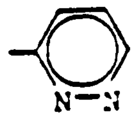 , 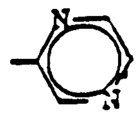 ,

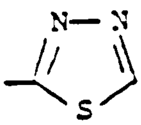 , 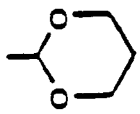 , 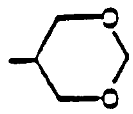 , 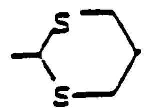 ,

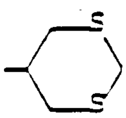 , 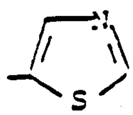 , 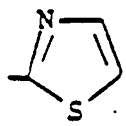 , 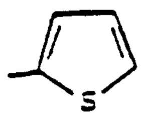 ,

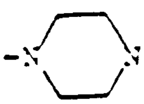 ,

$-A^3$

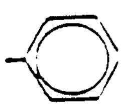 , 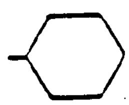 , 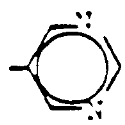 , 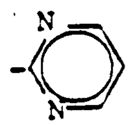 ,

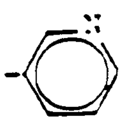 , 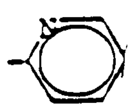 , 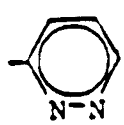

$-M^1$, $-M^2$ = $-CO-O$, $-O-CO$, $-CH_2O-$, $-OCH_2$
X = $-O-$.

**3.** The use as claimed in claim 2, wherein oxirane-2-carboxylic acid esters of the formula (IV) are used

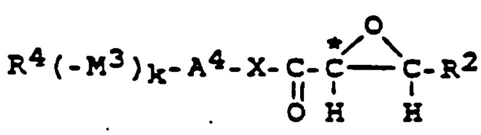 (IV)

where

$R^4$ denotes a straight-chain or branched alkyl or alkenyl radical containing 6 to 12 carbon atoms which may contain an asymmetric carbon atom;

$-M^3$ denotes $-O$, $-S$, $-O-CO$ or $-CO$

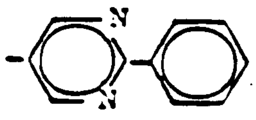 , 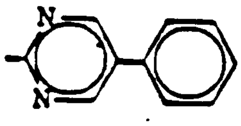 , 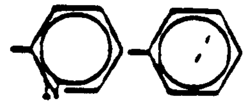 ,

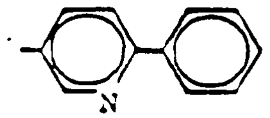 , 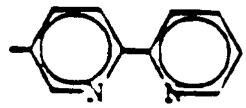 , 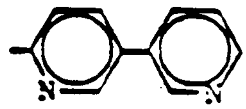 ,

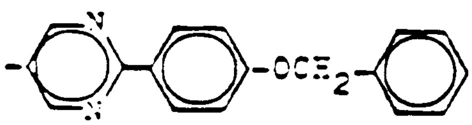 , 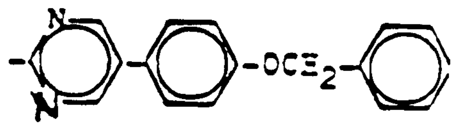 ,

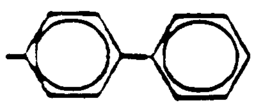 , 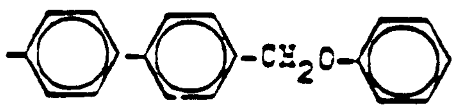 ,

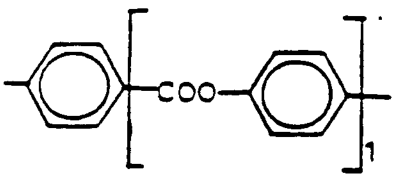 or 2

**4.** Optically active oxirane -2-carboxylic acid esters of the formula (I) as claimed in claim 2.

**5.** Optically active oxirane-2-carboxylic acid eaters of the formula (IV) as claimed in claim 3.

**6.** A liquid-crystal mixture which has a content of at least one optically active oxirane-2-carboxylic acid ester.

**7.** A liquid-crystal mixture which has a content of at least one optically active oxirane-2-carboxylic acid ester of the formula (I) as claimed in claim 2.

**8.** A liquid-crystal mixture which has a content of at least one optically active oxirane-2-carboxylic acid ester of the formula (IV) as claimed in claim 3.

**9.** An electrooptically switching or display element containing a liquid-crystal mixture as claimed in claim 6.

**10.** A process for the preparation of optically active oxirane-2-carboxylic acid esters of the formula (I) as claimed in claim 2, which comprises reacting mesogenic phenols of the formula (II) with derivatives of the oxirane-2-carboxylic acid of the formula (III), Y denoting an OH group or halogen:

$R^1(-A^1)_j(-M^1)_k(-A^2)_l(-M^2)_m(-A^3)_n-X-H$ (II)

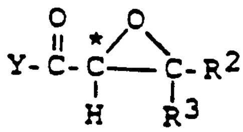

(III)

## Revendications

1. Utilisation d'esters d'acides oxiranne-2-carboxyliques, optiquement actifs, comme substances dopantes dans des mélanges à propriétés de cristaux liquides .

2. Utilisation selon la revendication 1, caractérisée en ce que l'on utilise les esters d'acides oxiranne-2-carboxyliques de formule générale (I)

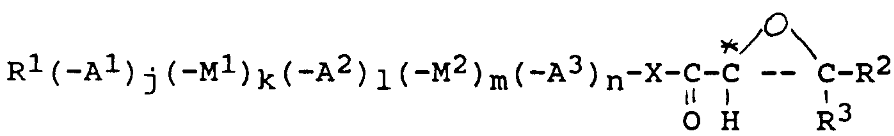

(I)

dans laquelle les symboles et indices ont le sens suivant :
$R^1$ représente

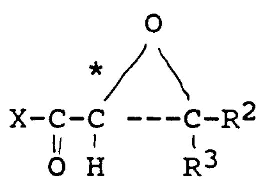

ou un reste alkyle, linéaire ou ramifié, ayant 1 à 16 atomes de carbone ou un reste alcényle linéaire ou ramifié ayant 3 à 16 atomes de carbone, ces restes pouvant eux-mêmes contenir des atomes de C asymétrique, un ou plusieurs groupes $-CH_2-$ non voisins pouvant être remplacés par -O-, -S-, -CO-, -O-CO- et/ou -CO-O-, et un ou plusieurs atomes de H pouvant être remplacés par F , Cl, Br;

$R^2$ représente H ou un groupe alkyle ayant 1 à 16 atomes de carbone ou un groupe cyclohexyle ou bicyclohexyle, qui peuvent également être substitués en position 4 ou 4', par une chaine alkyle ayant 1 à 16 atomes de carbone ;

$R^3$ représente H;

j et l valent 0,1 ou 2,

k et m valent 0 ou 1,

n vaut 0, 1 ou 2,

avec la réserve suivante : lorsque j et/ou l vaut 0, k vaut 0; lorsque n vaut O, m vaut 0, la somme (j+l+n) vaut au moins 1 et 3 au maximum;

-A1, -A2, représentent

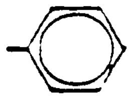 , 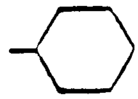 , 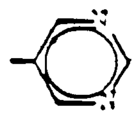 , 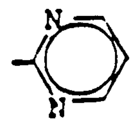 ,

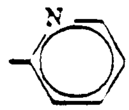 , 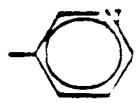 , 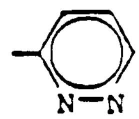 , 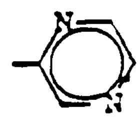 ,

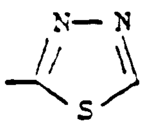 , 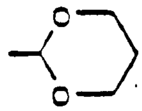 , 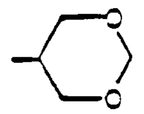 , 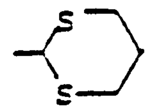 ,

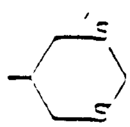 , 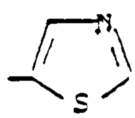 , 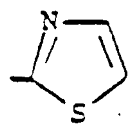 , 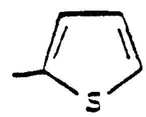 ,

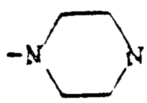 ,

– A3 représente

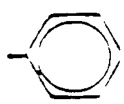 , 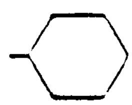 , 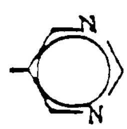 , 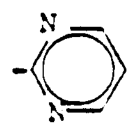 ,

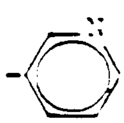 , 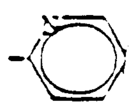 , 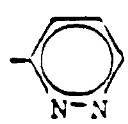

$-M^1$, $-M^2$ représentent – CO–O, –O–CO, $-CH_2O$, – OCH2
X représente O.

**3.** L'utilisation selon la revendication 2, caractérisée en ce qu'on utilise des esters d'acides oxiranne–2–carboxyliques de formule générale (IV)

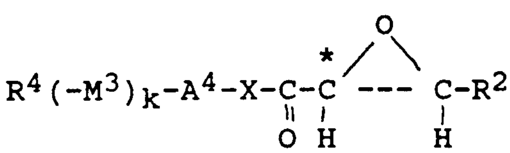 (IV)

dans laquelle

$R^4$ représente un reste alkyle ou alcényle, linéaire ou ramifié, ayant 6 à 12 atomes de carbone, qui peut contenir un atome de C asymétrique;

–M3 représente –O, –S, –O–CO ou –CO

–A4 représente

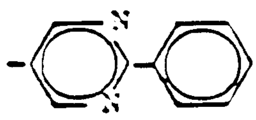 , 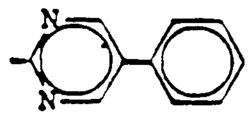 , 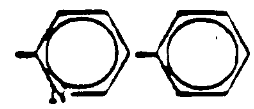 ,

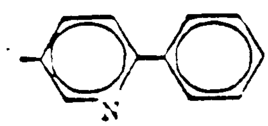 , 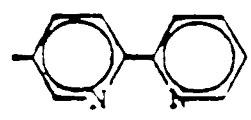 , 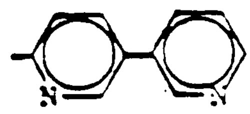 ,

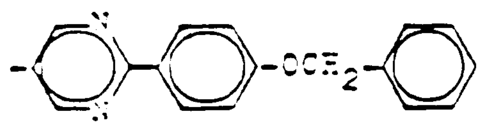 , 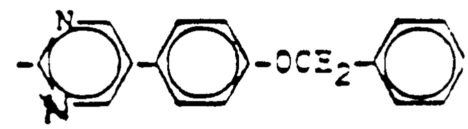 ,

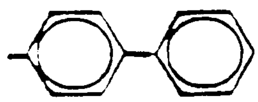 , 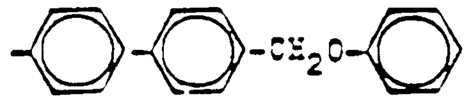 ,

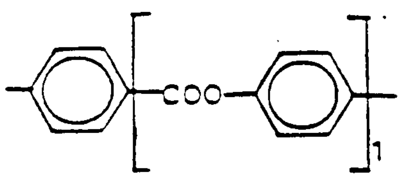 ou 2

**4.** Esters d'acides oxiranne-2-carboxyliques optiquement actifs de formule générale (I) selon la revendication 2.

**5.** Esters d'acides oxiranne-2-carboxyliques optiquement actifs, de formule générale (IV) selon la revendication 3.

**6.** Mélange à propriétés de cristaux liquides, caractérisé en ce qu'il contient au moins un ester d'acide oxiranne-2-carboxylique optiquement actif.

**7.** Mélange à propriétés de cristaux liquides, caractérisé en ce qu'il contient au moins un ester d'acide oxiranne-2-carboxylique, optiquement actif, de formule générale (I) selon la revendication 2.

**8.** Mélange à propriétés de cristaux liquides, caractérisé en ce qu'il contient au moins un ester d'acide oxiranne-2-carboxylique, optiquement actif, de formule générale (IV) selon la revendication 3.

**9.** Elément de commutation ou d'affichage électro-optique, contenant un mélange à propriétés de cristaux liquides selon la revendication 6.

**10.** Procédé pour préparer des esters d'acides oxiranne-2-carboxyliques optiquement actifs, de formule générale (I) selon la revendication 2, procédé caractérisé en ce qu'on fait réagir des phénols mésogènes de formule générale (II) avec des dérivés de l'acide oxiranne-2-carboxylique de formule générale (III) :

$$R^1(-A^1)_j(-M^1)_k(-A^2)_l(-M^2)_m(-A^3)n-X-H \qquad (II))$$

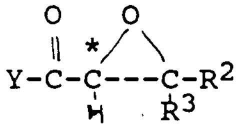

(III)

formules dans lesquelles Y représente un groupe OH ou un atome d'halogène.